# EUROPEAN PATENT SPECIFICATION

(11) **EP 3 965 592 B1**
(45) Date of publication and mention of the grant of the patent: **25.06.2025**
(21) Application number: 20730714.1
(22) Date of filing: 29.04.2020
(51) Int. Cl.: A23L 33/10, A61K 9/107, A23L 33/105, A23L 33/15, A23L 33/18, A61K 9/00, A61K 9/10

(54) **LIQUID COMPOSITION FOR THE ADMINISTRATION OF SUBSTANCES HAVING LOW SOLUBILITY AND LOW ENTERIC BIOAVAILABILITY**
FLÜSSIGE ZUSAMMENSETZUNG FÜR DIE VERABREICHUNG VON WIRKSTOFFEN MIT GERINGER LÖSLICHKEIT UND GERINGER BIOVERFÜGBARKEIT
COMPOSITION LIQUIDE POUR L'ADMINISTRATION DE COMPOSES AYANT UNE SOLUBILITE ET UNE BIODISPONIBILITE FAIBLES

(30) Priority: 06.05.2019 IT 201900006573
(43) Date of publication of application: 16.03.2022
(73) Proprietor: Gricar Chemical S.r.l., 20861 Brugherio MB (IT)
(72) Inventor: CARENZI, Gian Marco, 20861 Brugherio MB (IT); CONTINI, Marisa, 20861 Brugherio MB (IT)
(74) Representative: Perani & Partners S.p.A.
(86) International application number: PCT/IB2020/054019
(87) International publication number: WO 2020/225653

(56) References cited:
- US-A1- 2005 147 598
- US-A1- 2007 190 080
- US-A1- 2011 236 364
- US-A1- 2012 053 241
- US-A1- 2013 210 916
- US-A1- 2017 182 133

## Description

### FIELD OF THE INVENTION

The present invention relates to a composition in liquid form for vehiculating of active ingredients having low stability in water and/or low stability in gastric aqueous secretions and/or low intestinal uptake, and oral or sublingual formulations thereof,

### STATE OF THE ART

Nutraceutical formulations often experience the difficulty of making the active ingredients stable in both in liquid and solid form formulations and above all absorbable by the intestine, following oral intake. In particular, the uptake of lipophilic or slightly soluble substances in water through the enteric mucosa is strongly limited by the poor water solubility in the fluids flowing in the lumen. The crossing of the mucus layer that covers the enteric epithelium from the small intestine to the colon, called Unstirred Water Phase (UWP), in fact can retain these substances and slow them down preventing their contact with the enterocytes. In addition to these physical factors, there are other factors that can influence enteric absorption (enteric bioaccessibility), such as the presence of intercellular Tight Junctions that preclude the paracellular passage, in addition to the ATP-dependent outflow systems located in the enterocyte membrane such as the P-gP pump and the action of enteric cytochromes (CYP3A).

Taken together, these phenomena can make extremely difficult for many active ingredients extremely difficult to reach the bloodstream.

In order to overcome these physical, chemical and metabolic challenges, specific technologies have been developed which are able to increase the free diffusion of lipophilic and poorly soluble molecules in contact with enteric liquids. Technological forms such as nanoemulsions, nanocapsules, micellar dispersions, cyclodextrins and SEDDS (self-emulsifying delivery systems) have been the subject of chemical-pharmaceutical studies for decades in order to improve the oral bioavailability of many molecules of pharmaceutical interest and in recent years, also nutraceuticals.

Pharmaceutical compositions for the delivery of biologically active agents that are inactivated in the presence of water, are disclosed in e.g., US 2017/181133 A1.

Precisely in the nutraceutical sector, the growing need to develop increasingly high-performance formulations capable of ensuring the effective uptake of vitamins, vitamin-like factors, functional physiological or plant-derived substances has amplified the attention on the bioavailability of these substances and the need to develop technological forms capable of improving it. In the nutraceutical sector, substances such as Melatonin, Resveratrol, fat-soluble vitamins such as Vitamin A, D3 and K2 and the enzyme class, represent cornerstones for the protection of human health and well-being, due to the known and proven biological activities carried out and disease-prevention and physiological state-restoration properties. Because of these important health promotion and restoration properties, these substances, together with many others that characterize the sector of food supplements and nutraceuticals, are often the subject of clinical and in vitro studies on human tissue models, in order to assess their bioaccessibility and ultimately bioavailability following oral intake in humans. Many works published in the last 2 decades, unequivocally sanction as Melatonin, Resveratrol, various plant-origin substances and, although less significantly, some Vitamins such as D3 suffer from poor bioavailability following oral intake.

This pharmacological data is mainly attributable to the biophysical and metabolic phenomena described above.

Regarding the enzyme class, the main difficulty in guaranteeing their correct oral administration is making them to overcome the gastric barrier, with particular reference to the pH of around 1.5 on an empty stomach and the action of the proteolytic enzyme pepsin, precisely activated by acidic pH. Pepsin can quickly hydrolyze peptide chains of enzymes by completely or partially inactivating them. Furthermore, these substances are often not very compatible with the aqueous liquid forms in which they have low stability over time.

Last but not least, the vehiculation, especially in liquid form, of substances that have a low water stability can represent a difficult challenge due to the degradation and their reactivity with excipients of the formulation with relative loss of effectiveness of the preparation.

The sublingual route, already widely used in the pharmaceutical sector to ensure a rapid onset of the therapeutic effect and a reduced hepatic biotransformation (vascular bypass of the hepatic first-pass effect), may partly obviate the uptake obstacles mentioned above, but not to those of active ingredients stability, when in liquid form. A further limitation of such form of administration consists in low dosages, of the order of a few milligrams, compatible with sublingual route.

To date, therefore, especially in the nutraceutical sector, there is no form of liquid technological delivery intended for oral intake and able at the same time to guarantee a good stability of active ingredients that have a low stability in water and acidic gastric liquid and on the other hand to favor their bio-accessibility and that of active ingredients that have a low solubility and low uptake by the enteric epithelium.

### SUMMARY OF THE INVENTION

The applicant has now surprisingly found that it is possible to overcome the drawbacks of the state of the art by using, as a vehicle for these active ingredients, a liquid composition, in which the water is largely replaced by glycerol and in which the active ingredient is predispersed in a micellar form due to the presence of at least one medium- or long-chain fatty acid in liquid form in the presence of an alkalizing agent.

The subject of the present invention, which is defined by the claims, is a liquid composition comprising glycerol, at least one unsaturated fatty acid in liquid form, an alkalizing agent and water in which:
a) glycerol is present at concentrations comprised between 50% and 99% by weight, on the total weight of said liquid composition:
b) water at a concentration comprised between 0.01% and 35% by weight on the total weight of said liquid composition, and
wherein said alkalising agent is comprised at concentrations comprised between 1% and 5% by weight on the total weight of said composition.

Further objects of the present invention are therefore liquid oral and/or sublingual formulations comprising an active ingredient having low solubility and/or low stability in gastric secretion and/or low uptake by the intestine, and the liquid composition object of the present invention as a carrier.

A further object of the present invention is the process of preparing said oral or sublingual formulations which comprises the following stages:
i) Dissolving the alkalising agent in glycerol, if necessary, by heating at a temperature not higher than 70°C;
ii) separately dispersing the active ingredient into glycerol in case it is soluble therein or alternatively joining it to the micellar dispersion of the liquid-form fatty acid in water after alkalinisation thereof at 8.0<pH<9
iii) joining the two phases obtained in previous steps i) and ii)
iv) optionally adding to the formulation obtained at step iii) the solubilizer or co-emulsifier and/or at least one of the other known excipients selected from flavorings, sweeteners, preservatives, stabilizers, acidity correctors.

Finally, a further object of the present invention relates to the use of said formulations as food supplements, nutraceutical products, food for special medical purposes, baby food, medical devices.

The formulation for oral or sublingual administration object of the present invention is becoming immediately hydrated upon contact with aqueous solutions, such as for example body fluids such as saliva/gastric juices, enteric juices, giving rise to a clear and perfectly transparent micellar dispersion, as a proof of the effective hydro-dispersion of the active ingredient. This dispersion, by virtue of the partial or total neutralization of the gastric acid, carried out by the excess of alkalizing agent present, is not altered by the acidification by hydrochloric acid, which would displace the fatty acid sodium salt by destructuring the micellar dispersion itself. This phenomenon guarantees the integrity of the micellar system during gastric transit.

The micellar dispersion obtained with the oral/sublingual formulations object of the invention is also particularly advantageous in the case of the delivering peptides provided with enzymatic activity such as lactase, galactosidase, bromelain and others, for example. In fact, it is known that these substances are hydrolyzed by gastric pepsin in the presence of acidic pH and that such gastric pepsin is no longer active at pH values > 5 (Margareth R. C. Marques Enzymes in the Dissolution Testing of Gelatin Capsules. AAPS PharmSciTech. 2014 Dec; 15(6): 1410-1416). Such a feature allows for the active ingredients labile in acidic pH and in contact with pepsin not to be degraded during the average gastric emptying time which is estimated to be 90 minutes. Furthermore, the prevailing glycerol component of the system also ensures that long-lasting chemical stability of substances labile in water, including the enzymes themselves, is maintained.

Finally, the oral or sublingual formulations object of the invention, when they come into contact with body fluids, instantly forming a micellar formulation, are able to render the lipophilic active ingredients easily water dispersible.

### DETAILED DESCRIPTION OF THIS INVENTION

For the purposes of the present invention, the phrase composition.../formulation... "comprising" does not exclude the possibility that there are additional components in addition to those expressly listed.

For the purposes of the present invention, systemic oral formulation means a systemic formulation assimilable by hepatic bypass.

The liquid compositions object of the present invention preferably contain glycerol in concentrations comprised between 65% and 85% by weight on the total weight of said composition.

According to another preferred embodiment, the same contain the water in concentrations comprised between 10 and 30% by weight on the weight of said liquid composition.

For the purposes of the present invention, by unsaturated linear unsaturated liquid fatty acid is meant preferably a medium- or long-chain fatty acid containing at least one ethylenic unsaturation, where by medium chain is meant a linear or branched C8-C17 alkyl chain, by long chain is meant a straight or branched C18-C24 alkyl chain.

Still more preferably said medium- or long-chain fatty acid, containing at least one ethylene unsaturation, is selected from: conjugated linoleic acid, linolenic acid, eicosapentaenoic acid, docosahexaenoic acid.

Said unsaturated linear fatty acid is preferably contained in quantities preferably comprised between 0.01% and 5% by weight, preferably between 0.50 and 2% by weight.

The alkalizing agent is preferably selected from alkaline or alkaline earth metal hydroxides, sodium carbonate, sodium bicarbonate and mixtures thereof, preferably it is sodium bicarbonate.

This component is contained in an amount comprised between 1% and 5% by weight on the total weight of said liquid composition.

The liquid composition object of the present invention can optionally contain an emulsified or solubilized agent, preferably selected from: polyoxyethylene sorbitan esters, according to a particularly preferred solution it is polysorbate 80, sucrose esters, egg lecithin, soy or sunflower.

Preferably said emulsified or solubilized agent is contained in the liquid compositions object of the present invention in quantities ranging from 0.01% to 5% by weight on the total weight of said composition.

The active ingredient having low slightly soluble and/or not very stable in gastric secretions and/or poorly absorbable by the intestine contained in the oral or sublingual liquid formulations further object of the present invention is preferably chosen from: in at least one of the following categories: amino acids and derivatives, oligopeptides, polypeptides, enzymes, steroid terpenes, saponins, stilbenes, fat-soluble vitamins, water-soluble vitamins, vitamin-like factors, flavonoids, polyphenols.

The oral or sublingual formulations object of the present invention can optionally comprise at least one of the known excipients selected from sweeteners, flavoring substances, acidity correctors, preservative stabilizers.

Some examples of oral or sublingual liquid formulation object of the present invention and the related preparation processes, as well as an experimental simulation test of the neutralization of gastric juices obtained with the liquid formulation of Example 3 object of the present invention, are reported below for illustrative but not limitative purposes.

### Example 1

| | |
|---|---|
| Vitamin D3 (oil 1.000.000 IU/gr) | 0.05 |
| Glycerol | 70.0 |
| Polysorbate 80 | 3.00 |
| Conjugated Linoleic Acid (CLA) | 0.60 |
| Sodium bicarbonate | 1.20 |
| Sucralose | 0.02 |
| Vanilla flavor | 0.12 |
| Water | Qb 100 gr |

### Method

Disperse Vitamin D3 in Polysorbate 80 and add CLA. Dissolve sodium bicarbonate in Glycerol by heating at 50 ° C. Dissolve Sucralose and vanilla flavor in water. Combine the two phases thus obtained by mixing with a mechanical stirrer until a homogeneous and clear phase is obtained.

### Example 2

| | |
|---|---|
| Melatonin | 0.06 |
| Glycerol | 80.0 |
| Polysorbate 80 | 1.00 |
| Conjugated Linoleic Acid (CLA) | 0.80 |
| Sodium bicarbonate | 1.80 |
| Sucralose | 0.02 |
| Vanilla flavor | 0.12 |
| Water | Qb 100 g |

### Method

Combine CLA with Polysorbate 80. Dissolve melatonin in glycerol by heating at 50 ° C. Dissolve sodium bicarbonate in the available water until completely dissolved (heat to 45 ° C) and then dissolve Sucralose and vanilla flavor. Combine the two phases thus obtained by mixing with a mechanical stirrer until a homogeneous and clear phase is obtained.

### Example 3

| | |
|---|---|
| Lactase (100,000 IU/g) | 0.75 |
| Glycerol | 70.0 |
| Conjugated Linoleic Acid (CLA) | 0.80 |
| Sodium bicarbonate | 4.00 |
| Sucralose | 0.02 |
| Vanilla flavor | 0.12 |
| Water | Qb 100 g |

### Method

Combine CLA with Polysorbate 80. Dissolve baking soda in Glycerol by heating at 50 ° C. Dissolve Lactase in the available water until completely dispersed and then dissolve Sucralose and vanilla flavor. Combine the two phases thus obtained by mixing with a mechanical stirrer until a homogeneous and clear phase is obtained.

### Example 4- Experimental simulation of gastric pH neutralization on an empty stomach

40 ml of fasted simulated gastric liquid (FaSSGF) composed of demineralized water, sodium chloride (34.2 mM) and diluted hydrochloric acid (sol. 2% w/v) up to pH = 1.5 was placed in a 50 ml beaker and subjected to magnetic stirring at 200 rpm. The pH meter probe was then introduced, and the continuous pH measurement was set. The FaSSGF was thermostated at 37 ° C. 1 ml of the formulation corresponding to the composition described in Example 3 was then added to the FaSSGF at 37 ° C and under stirring. The system pH rises rapidly to 8.9 (45 seconds). After adding a total of 12 ml of diluted Hydrochloric acid. (sol. 2% w/v) over 90 minutes (every 15 minutes) to the simulate gastric acid secretion, the system pH settled down at 5.2.

## Claims

1. A liquid composition comprising glycerol, at least an unsaturated fatty acid in liquid form, an alkalising agent and water wherein:
a) glycerol is present at concentrations comprised between 50% and 99% by weight, on the total weight of said liquid composition:
b) water at a concentration comprised between 0.01% and 35% by weight on the total weight of said liquid composition, and
wherein said alkalising agent is comprised at concentrations comprised between 1% and 5% by weight on the total weight of said composition.

2. The liquid composition according to claim 1, wherein glycerol is contained at concentrations comprised between 65% and 85% by weight on the total weight of said composition.

3. The liquid composition according to claim 1 or 2, wherein water is contained at concentrations comprised between 10 and 30% by weight on the weight of said liquid composition.

4. The liquid composition according to any one of claims 1-3, wherein the unsaturated fatty acid in liquid form is a long- or medium-chain fatty acid containing at least an ethylene unsaturation, preferably selected from: oleic acid, linoleic acid, conjugated Linoleic acid, Linolenic Acid, eicosapentaenoic acid, docosahexaenoic acid.

5. The liquid composition according to any one of claims 1-4, wherein said fatty acid in liquid form is present in amounts comprised between 0.01% and 5% by weight, preferably between 0.50 and 2% by weight.

6. The liquid composition according to any one of claims 1-5, wherein the alkalising agent is selected from: alkaline or alkaline earth metal hydroxides, sodium carbonate, sodium bicarbonate and relative mixtures, preferably it is sodium bicarbonate.

7. The liquid composition according to any one of claims 1 to 6, comprising an emulsifier or solubilizing agent, preferably selected from: polyoxyethylene sorbitan esters, sucrose esters, egg, soya or sunflower lecithin.

8. The liquid composition according to claim 7, wherein said co-emulsifier or solubilizing agent is present at concentrations comprised between 0.01% and 5% by weight on the total weight of said composition, preferably between 1 and 4 by weight on the total weight of said formulation.

9. Oral or sublingual formulations in liquid form comprising an active ingredient poorly soluble and/or poorly stable in gastric secretions and/or poorly absorbable by the bowel and comprising as a vehicle the liquid composition according to any one of claims 1 to 8.

10. The oral or sublingual formulations according to claim 9, wherein said one active ingredient poorly soluble and/poorly stable in gastric secretions and/or poorly absorbable by the bowel is selected from at least one of the following categories: amino acids and derivatives, oligopeptides, polypeptides, enzymes, steroid terpenes, saponins, stilbenes, fat-soluble vitamins, water-soluble vitamins, vitamin-like factors, flavonoids, polyphenols.

11. The oral and/or sublingual formulations according to any one of claims 9 and 10 comprising at least one of the known excipients selected from sweetening substances, flavoring substances, acidity correctors, preservative stabilizers.

12. A process for preparing oral or sublingual formulations according to any one of claims 9-11 comprising the following steps:
i) Dissolving the alkalising agent in glycerol, if necessary, by heating at a temperature not higher than 70°C;
ii) separately dispersing the active ingredient into glycerol in case it is soluble therein or alternatively joining it to the micellar dispersion of the liquid-form fatty acid in water after alkalinisation thereof at 8.0<pH<9
iii) joining the two phases obtained in previous steps i) and ii)
iv) optionally adding to the formulation obtained at step iii) the solubilizer or co-emulsifier and/or at least one of the other known excipients selected from flavorings, sweeteners, preservatives, stabilizers, acidity correctors.

13. The oral or sublingual formulations according to any one of claims 9 to 11, in the form of food supplements, nutraceutical products, food for special medical purposes, baby food, medical devices.

## Patentansprüche

1. Flüssige Zusammensetzung, die Glycerin, mindestens eine ungesättigte Fettsäure in flüssiger Form, ein Alkalisierungsmittel und Wasser enthält, wobei:
a) Glycerin in Konzentrationen zwischen 50 und 99 Gew.- %, bezogen auf das Gesamtgewicht der flüssigen Zusammensetzung, vorhanden ist:
b) Wasser in einer Konzentration zwischen 0,01 und 35 Gew.- %, bezogen auf das Gesamtgewicht der flüssigen Zusammensetzung, und
wobei das Alkalisierungsmittel in Konzentrationen zwischen 1 und 5 Gew.- %, bezogen auf das Gesamtgewicht der Zusammensetzung, enthalten ist.

2. Flüssige Zusammensetzung nach Anspruch 1, wobei Glycerin in Konzentrationen zwischen 65 und 85 Gew.-%, bezogen auf das Gesamtgewicht der Zusammensetzung, enthalten ist.

3. Flüssige Zusammensetzung nach Anspruch 1 oder 2, wobei Wasser in Konzentrationen zwischen 10 und 30 Gew.-%, bezogen auf das Gewicht der flüssigen Zusammensetzung, enthalten ist.

4. Flüssige Zusammensetzung nach einem der Ansprüche 1 bis 3, wobei die ungesättigte Fettsäure in flüssiger Form eine lang- oder mittelkettige Fettsäure ist, die mindestens eine Ethylen-Ungesättigtheit enthält, vorzugsweise ausgewählt aus: Ölsäure, Linolsäure, konjugierter Linolsäure, Linolensäure, Eicosapentaensäure, Docosahexaensäure.

5. Flüssige Zusammensetzung nach einem der Ansprüche 1-4, wobei die Fettsäure in flüssiger Form in Mengen zwischen 0,01 und 5 Gew.-%, vorzugsweise zwischen 0,50 und 2 Gew.-%, vorhanden ist.

6. Flüssige Zusammensetzung nach einem der Ansprüche 1-5, wobei das Alkalisierungsmittel ausgewählt ist aus: Alkali- oder Erdalkalimetallhydroxiden, Natriumcarbonat, Natriumbicarbonat und relativen Mischungen, vorzugsweise ist es Natriumbicarbonat.

7. Flüssige Zusammensetzung nach einem der Ansprüche 1 bis 6, umfassend einen Emulgator oder Solubilisierungsmittel, vorzugsweise ausgewählt aus: Polyoxyethylensorbitanestern, Saccharoseestern, Ei, Soja oder Sonnenblumenlecithin.

8. Flüssige Zusammensetzung nach Anspruch 7, wobei der Co-Emulgator oder das Solubilisierungsmittel in Konzentrationen zwischen 0,01 und 5 Gew.-%, bezogen auf das Gesamtgewicht der Zusammensetzung, vorzugsweise zwischen 1 und 4 Gew.-%, bezogen auf das Gesamtgewicht der Formulierung, vorhanden ist.

9. Orale oder sublinguale Formulierungen in flüssiger Form, die einen in Magensekreten schlecht löslichen und/oder schlecht stabilen und/oder vom Darm schlecht resorbierbaren Wirkstoff umfassen und als Vehikel die flüssige Zusammensetzung nach einem der Ansprüche 1 bis 8 umfassen.

10. Orale oder sublinguale Formulierungen nach Anspruch 9, wobei der eine in Magensekreten schlecht lösliche und/oder schlecht stabile und/oder vom Darm schlecht resorbierbare Wirkstoff aus mindestens einer der folgenden Kategorien ausgewählt ist: Aminosäuren und Derivaten, Oligopeptiden, Polypeptiden, Enzymen, Steroidterpenen, Saponinen, Stilbenen, fettlöslichen Vitaminen, wasserlöslichen Vitaminen, vitaminähnlichen Faktoren, Flavonoiden, Polyphenolen.

11. Orale und/oder sublinguale Formulierungen nach einem der Ansprüche 9 und 10, umfassend mindestens einen der bekannten Hilfsstoffe, ausgewählt aus Süßungsmitteln, Aromastoffen, Säurekorrektoren und Konservierungsstabilisatoren.

12. Verfahren zur Herstellung von oralen oder sublingualen Formulierungen nach einem der Ansprüche 9-11, umfassend die folgenden Schritte:
i) Auflösen des Alkalisierungsmittels in Glycerin, gegebenenfalls durch Erhitzen auf eine Temperatur von nicht mehr als 70 °C;
ii) getrenntes Dispergieren des Wirkstoffs in Glycerin, falls er darin löslich ist, oder alternativ dessen Zusammenführen mit der mizellaren Dispersion der Fettsäure in flüssiger Form in Wasser nach dessen Alkalisierung bei 8,0<pH<9
iii) Zusammenführen der zwei in den vorherigen Schritten i) und ii) erhaltenen Phasen
iv) gegebenfalls Zugeben des Lösungsvermittlers oder Co-Emulgators und/oder mindestens eines der anderen bekannten Hilfsstoffe, ausgewählt aus Aromastoffen, Süßstoffen, Konservierungsmitteln, Stabilisatoren, Säurekorrektoren, zu der in Schritt iii) erhaltenen Formulierung.

13. Orale oder sublinguale Formulierungen nach einem der Ansprüche 9 bis 11 in Form von Nahrungsergänzungsmitteln, nutrazeutischen Produkten, Lebensmitteln für besondere medizinische Zwecke, Babynahrung, medizinischen Geräten.

## Revendications

1. Composition liquide comprenant du glycérol, au moins un acide gras insaturé sous forme liquide, un agent alcalinisant et de l'eau dans laquelle :
a) le glycérol est présent à des concentrations comprises entre 50 % et 99 % en poids, par rapport au poids total de ladite composition liquide :
b) de l'eau à une concentration comprise entre 0,01 % et 35 % en poids par rapport au poids total de ladite composition liquide, et
dans laquelle ledit agent alcalinisant est compris dans des concentrations comprises entre 1 % et 5 % en poids par rapport au poids total de ladite composition.

2. Composition liquide selon la revendication 1, dans laquelle le glycérol est contenu à des concentrations comprises entre 65 % et 85 % en poids par rapport au poids total de ladite composition.

3. Composition liquide selon la revendication 1 ou 2, dans laquelle l'eau est contenue à des concentrations comprises entre 10 et 30 % en poids par rapport au poids de ladite composition liquide.

4. Composition liquide selon l'une des revendications 1 à 3, dans laquelle l'acide gras insaturé sous forme liquide est un acide gras à chaîne longue ou moyenne contenant au moins une insaturation éthylénique, de préférence choisi parmi : l'acide oléique, l'acide linoléique, l'acide linoléique conjugué, l'acide linolénique, l'acide eicosapentaénoïque, l'acide docosahexaénoïque.

5. Composition liquide selon l'une des revendications 1 à 4, dans laquelle ledit acide gras sous forme liquide est présent en quantités comprises entre 0,01 % et 5 % en poids, de préférence entre 0,50 et 2 % en poids.

6. Composition liquide selon l'une des revendications 1 à 5, dans laquelle l'agent alcalinisant est choisi parmi les hydroxydes de métaux alcalins ou alcalino-terreux, le carbonate de sodium, le bicarbonate de sodium et les mélanges correspondants, de préférence le bicarbonate de sodium.

7. Composition liquide selon l'une des revendications 1 à 6, comprenant un émulsifiant ou un agent solubilisant, de préférence choisi parmi : les esters de sorbitan polyoxyéthylénés, les esters de saccharose, la lécithine d'œuf, de soja ou de tournesol.

8. Composition liquide selon la revendication 7, dans laquelle ledit coémulsifiant ou agent solubilisant est présent à des concentrations comprises entre 0,01 % et 5 % en poids par rapport au poids total de ladite composition, de préférence entre 1 et 4 en poids par rapport au poids total de ladite formulation.

9. Formulations orales ou sublinguales sous forme liquide comprenant un principe actif peu soluble et/ou peu stable dans les sécrétions gastriques et/ou peu absorbable par l'intestin et comprenant comme véhicule la composition liquide selon l'une quelconque des revendications 1 à 8.

10. Formulations orales ou sublinguales selon la revendication 9, dans lesquelles ledit principe actif peu soluble et/ou peu stable dans les sécrétions gastriques et/ou peu absorbable par l'intestin est choisi dans au moins une des catégories suivantes : acides aminés et dérivés, oligopeptides, polypeptides, enzymes, terpènes stéroïdiens, saponines, stilbènes, vitamines liposolubles, vitamines hydrosolubles, facteurs similaires aux vitamines, flavonoïdes, polyphénols.

11. Formulations orales et/ou sublinguales selon l'une des revendications 9 et 10 comprennent au moins un des excipients connus choisis parmi les substances sucrantes, les substances aromatiques, les correcteurs d'acidité, les stabilisateurs de conservation.

12. Procédé de préparation de formulations orales ou sublinguales selon l'une des revendications 9 à 11, comprenant les étapes suivantes :
i) dissoudre l'agent alcalinisant dans le glycérol, si nécessaire, en le chauffant à une température ne dépassant pas 70 °C ;
ii) disperser séparément l'ingrédient actif dans le glycérol s'il y est soluble ou en le joignant à la dispersion micellaire de l'acide gras sous forme liquide dans l'eau après alcalinisation de celle-ci à 8,0<pH<9
iii) joindre les deux phases obtenues aux étapes i) et ii) précédentes
iv) ajouter éventuellement à la formulation obtenue à l'étape iii) le solubilisant ou le co-émulsifiant et/ou au moins l'un des autres excipients connus choisis parmi les arômes, les édulcorants, les conservateurs, les stabilisateurs, les correcteurs d'acidité.

13. Formulations orales ou sublinguales selon l'une des revendications 9 à 11, sous forme de compléments alimentaires, de produits nutraceutiques, d'aliments destinés à des fins médicales spéciales, d'aliments pour bébés, de dispositifs médicaux.
